(19) **Europäisches Patentamt / European Patent Office / Office européen des brevets**

(11) **EP 4 470 566 A1**

(12) # EUROPEAN PATENT APPLICATION

(43) Date of publication:
**04.12.2024  Bulletin 2024/49**

(21) Application number: **23176898.7**

(22) Date of filing: **02.06.2023**

(51) International Patent Classification (IPC):
**A61K 47/02** *(2006.01)*    **A61K 9/00** *(2006.01)*
**A61K 31/137** *(2006.01)*    **A61K 47/26** *(2006.01)*
**A61K 47/20** *(2006.01)*    **A61K 47/12** *(2006.01)*
**A61K 47/18** *(2017.01)*

(52) Cooperative Patent Classification (CPC):
**A61K 9/0019; A61K 31/137; A61K 47/02;
A61K 47/12; A61K 47/26;** A61K 47/183;
A61K 47/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **B. Braun Melsungen AG**
**34212 Melsungen (DE)**

(72) Inventors:
• **REDONDO, Elena**
  **34212 Melsungen (DE)**
• **LÓPEZ, Miriam**
  **34212 Melsungen (DE)**

• **FERNANDEZ, Roberto**
  **34212 Melsungen (DE)**
• **PLANAS, Laura**
  **34212 Melsungen (DE)**
• **ÁLVAREZ, Ana Belen**
  **34212 Melsungen (DE)**
• **TIMONEDA RAMIA, Cristina**
  **34212 Melsungen (DE)**
• **FISCHER, Oliver**
  **34212 Melsungen (DE)**

(74) Representative: **Maiwald GmbH**
**Elisenhof**
**Elisenstraße 3**
**80335 München (DE)**

(54) **ROOM TEMPERATURE STABLE FORMULATION OF NOREPINEPHRINE**

(57)    The present disclosure relates to a container comprising a liquid phase and a gas phase, wherein the liquid phase is a pharmaceutical composition comprising norepinephrine, or a pharmaceutically acceptable salt thereof, a buffering system consisting of citric acid and a pharmaceutically acceptable salt thereof, at least one tonicity agent, and water; and wherein the gas phase has an oxygen content of 0.2 % (V/V) or more based on the total volume of the gas phase; and a method of manufacturing the same.

EP 4 470 566 A1

## Description

### Field of the invention

The present invention relates to a container comprising a liquid phase comprising norepinephrine, or a pharmaceutically acceptable salt thereof, and a gas phase having an oxygen content of 0.2 % (V/V) or more, as well as to a method of manufacturing said container.

### Background

Norepinephrine (NE), also called norepinephrine or noradrenaline (NA), is an organic chemical in the catecholamine family that functions in the brain and body as both a hormone and neurotransmitter. "Norepinephrine" is also the international nonproprietary name given to the drug.

Norepinephrine itself is classified as a sympathomimetic drug and is often used as an emergency medicine by intravenous injection: its effects are the increase of heart rate, as well as cardiac contractility, and constriction of blood vessels by binding to alpha and beta adrenoceptors. Therefore, norepinephrine is very useful for treating medical emergencies that involve critically low blood pressure, such as shock due to heart problems (cardiogenic shock), uncontrolled bleeding (hypovolemic shock), severe infections (septic shock), allergic reactions (anaphylactic shock), or due to damage to the nervous system (neurogenic shock).

Shock is a life-threatening condition that occurs when the body is not getting enough blood flow. Lack of blood flow means that the cells and organs do not get enough oxygen and nutrients to function properly. Many organs can be damaged as a result. Shock requires immediate treatment and can get worse very rapidly. Therefore, lethality is high, especially when the shock is not treated rapidly and adequately.

Hence, pharmaceutical companies are facing the challenge of providing formulations of norepinephrine that are ready-to-inject, because medical emergencies do not allow for the time to freshly prepare an injectable formulation, e.g. by reconstitution of a lyophilized composition. Furthermore, the provision of a ready-to-inject formulation increases medication safety and lowers the preparation effort of the medication by nurses or other medical practitioners.

However, liquid, ready-to-inject formulations of norepinephrine and its pharmaceutically acceptable salts suffer from high instability, that is that norepinephrine rapidly decomposes through chemical reactions. In particular, norepinephrine is susceptible to oxidation reactions and racemization: Specifically, the formation of noradrenolone is a commonly known problem. Noradrenolone is formed through oxidation of the benzyl alcohol group of norepinephrine to the benzaldehyde derivative, that is noradrenolone. Therefore, ready-to-inject formulations of norepinephrine usually contain organic compounds with antioxidant properties and require a preparation and packaging under strict exclusion of oxygen.

For instance, the marketed norepinephrine formulation Levophed® of Hospira contains high amounts of sodium metabisulphite in a concentration of 0.2 mg/mL to prevent oxidation. However, the use of sulfite, metasulfite, or other antioxidants bears the risk of (further) allergic reactions in the patient upon intravenous administration which may not only reduce any beneficial effect of norepinephrine but can even result in a further deterioration of the health status of the patient who is already in need of intensive care.

Another option of stabilizing norepinephrine in a liquid formulation is the manufacturing and packing under strict oxygen-free conditions, thus reducing the amount of oxygen in the formulations to 100 ppb (part per billion) or even lower. However, such manufacturing process is highly complex and, therefore, involves high costs.

Other commercially available products of norepinephrine use a chelating agents, such as high amounts of EDTA, to stabilize norepinephrine (e.g. "Noradrenalin Aguettant").

However, most - if not all - ready-to-inject norepinephrine formulations lack storage stability and should therefore be discarded within one day after opening when stored at room temperature.

### Summary

It has now surprisingly been found by the inventors of the present disclosure that the presence of antioxidants in norepinephrine formulations when used in concentrations effective to inhibit the oxidation of norepinephrine cause other side reactions resulting in the formation of other, unknown impurities as well as to higher levels of degradation as measured by assay during sterilization and storage. This is, for instance, shown in example 3 of the present disclosure. Therefore, there is still need for norepinephrine formulations that are storage stable, contain only pharmaceutically acceptable, and chemically inert or non-reactive substances. Advantageously, such compounds do not produce adverse effects to the patient, e.g. endogenous compounds that are naturally produced in the human body.

In view of the above, there is still a need for improved stable, low concentration, and antioxidant free ready-to-inject formulations of norepinephrine that can be stored at ambient or room temperature, as well as easy, cheap, and convenient methods of manufacturing the same. Specifically, it is desirable for such formulation of norepinephrine to be stable at a temperatures of about 25 °C for a period of one year or more, even when oxygen is present in the container comprising such formulation. At the same time, it is desirable that such stable formulation can be conveniently prepared without the necessity of oxygen control, such as under oxygen reduced atmosphere, during production.

The inventors of the present disclosure have surprisingly found that room temperature stable compositions of norepinephrine can be prepared without using an antioxidant agent by using a specific buffering system consisting of citric acid and a pharmaceutically acceptable salt thereof. When such formulation is placed in a container, the pharmaceutical composition remains stable, i.e. the norepinephrine does not or substantially not decrease over time, even in the presence of oxygen in the supernatant gas phase.

According to a first aspect, the present disclosure relates to a container comprising a liquid phase and a gas phase, wherein

a) the liquid phase is a pharmaceutical composition comprising

i) norepinephrine, or a pharmaceutically acceptable salt thereof, in a concentration of from about 0.3 mM to about 12 mM, and
ii) a buffering system consisting of citric acid and a pharmaceutically acceptable salt thereof, wherein the buffering system is present in a concentration in the range of from 1 mM to 30 mM; and
iii) at least one tonicity agent; and
iv) water,

wherein the liquid phase has a pH in the range of from about 3.0 to about 4.0; and,
b) the gas phase has an oxygen content of 0.2 % (V/V) or more based on the total volume of the gas phase.

According to a second aspect, the present disclosure relates to a method for manufacturing a container according to the first aspect of the present disclosure comprising the steps of

a) dissolving a tonicity agent, a buffering system and norepinephrine or pharmaceutically acceptable salt thereof in water;
b) adjusting the pH of the solution obtained from step a);
c) filtering the resulting norepinephrine solution obtained from step b);
d) filling the solution obtained from step c) into a container;
e) flushing said container with nitrogen to adjust the oxygen content in the gas phase;
wherein at least one of steps a) to d) is carried out under an atmosphere comprising at least 1 % (V/V) of oxygen.

## Detailed Description

The invention of the present disclosure is described in the following in more detail, exemplified by preferred embodiments and embodiment examples. However, it is understood that the scope of the present disclosure is not limited thereto, but only by the appendant claims.

The present disclosure, in very general terms, relates to two aspects, namely a first aspect being directed to a container, and a second aspect being directed to a method of manufacturing.

The container

According to a first aspect, the present disclosure relates to a container comprising a liquid phase and a gas phase, wherein

a) the liquid phase is a pharmaceutical composition comprising norepinephrine, or a pharmaceutically acceptable salt thereof, in a concentration of from about 0.3 mM to about 12 mM, and a buffering system consisting of citric acid and a pharmaceutically acceptable salt thereof, wherein the buffering system is present in a concentration in the range of from 1 mM to 30 mM; and at least one tonicity agent; and water, wherein the liquid phase has a pH in the range of from about 3.0 to about 4.0; and,
b) the gas phase has an oxygen content of 0.2 % (V/V) or more, based on the total volume of the gas phase.

The inventors of the present disclosure have surprisingly found that a buffering composition consisting of citric acid and a pharmaceutically acceptable salt thereof are suitable to stabilize norepinephrine, or a pharmaceutically acceptable salt thereof, without the need of antioxidative substances. Even more strikingly, the norepinephrine, or pharmaceutically acceptable salt thereof, is stable, even in the presence of oxygen. Therefore, the buffering composition enables the production of ready-to-inject formulations under less strict control of the oxygen content of the atmosphere, and hence cheaper and more convenient production.

According to another preferred embodiment, the container is a sealed container. According to a preferred embodiment of the present disclosure, the container is a glass vial. When the container is a glass vial, the glass vial may be sealed with a rubber stopper. It is understood that rubber stoppers may allow oxygen to come into the container when stored for a longer period of time. In other words, the oxygen content in the gas phase increases over time of storage. Nevertheless, the inventors of the present disclosure have surprisingly found that the buffering system according to the present disclosure even then stabilizes the norepinephrine formulation, i.e. the norepinephrine does not decompose rapidly.

According to a further preferred embodiment of the present disclosure, the container is a sealed glass vial. According to a further preferred embodiment, the container is a glass ampoule. According to another preferred embodiment of the present disclosure, the container is an IV bag.

According to another preferred embodiment, the container is a prefilled syringe. When the container is a prefilled syringe, the prefilled syringe is, according to a further preferred embodiment of the present disclosure, placed within secondary container, the secondary container optionally comprising an oxygen scavenger. According to a further preferred embodiment, the secondary container is at least partially polymeric. According to a further preferred embodiment, the secondary container is a plastic bag. According to a further preferred embodiment, the secondary container is an aluminium overwrap.

The liquid phase

According to the present disclosure, the liquid phase is a pharmaceutical composition. In other words, the liquid phase is a pharmaceutical formulation that contains only pharmaceutically acceptable ingredients in pharmaceutically acceptable amounts. According to a preferred embodiment of the present disclosure, the liquid phase is suitable for parenteral administration. According to a further preferred embodiment of the present disclosure, the liquid phase is suitable for intravenous administration.

According to another preferred embodiment of the present disclosure, the liquid phase is sterile. According to a further preferred embodiment of the present disclosure, the liquid phase comprised in the container is sterilized by filtering and aseptic filling. According to a further preferred embodiment of the present disclosure, the liquid phase comprised in the container is sterilized by filtering and thermal sterilization. According to another preferred embodiment of the present disclosure, the liquid phase comprised in the container is sterilized by thermal sterilization.

According to the present disclosure, the liquid phase has a pH in the range of from about 3.0 to about 4.0.

According to a preferred embodiment of the present disclosure, the liquid phase has a pH in the range of from about 3.2 to about 3.8. According to another preferred embodiment of the present disclosure, the liquid phase has a pH in the range of from about 3.0 to about 3.6. According to another preferred embodiment of the present disclosure, the liquid phase has a pH in the range of from about 3.2 to about 3.8. According to another preferred embodiment of the present disclosure, the liquid phase has a pH in the range of from about 3.2 to about 3.5. According to another preferred embodiment of the present disclosure, the liquid phase has a pH in the range of from about 3.3 to about 3.45. According to a further preferred embodiment of the present disclosure, the liquid phase has a pH in the range more preferably of from about 3.35 to about 3.45. According to a most preferred embodiment of the present disclosure, the liquid phase has a pH of about 3.4.

The inventors of the present disclosure have surprisingly found that norepinephrine is particularly stable at such pH as evidenced in examples 1-5 of the present disclosure. In other words, the pH in the range of from about 3.0 to about 4.0, with the further preferred embodiments, increases the stability of the norepinephrine in the liquid phase.

According to a preferred embodiment of the present disclosure, the norepinephrine is the *R*-enantiomer of norepinephrine. It is understood that the *R*-enantiomer of norepinephrine is the naturally occurring enantiomer of norepinephrine, that is produced in the adrenal glands. Therefore, it is understood that *R*-norepinephrine is the active isomer of norepinephrine. It is thus preferred to use the *R*-enantiomer of norepinephrine.

According to another preferred embodiment of the present disclosure, the norepinephrine is a mixture of *R*-enantiomer and *S*-enantiomer of norepinephrine.

According to a further preferred embodiment of the present disclosure, the *R*-enantiomer of norepinephrine is present in an enantiomeric excess (ee) of 30 % or more. According to a further preferred embodiment of the present disclosure, the *R*-enantiomer of norepinephrine is present in an enantiomeric excess (ee) of 40 % or more. According to a further preferred embodiment of the present disclosure, the *R*-enantiomer of norepinephrine is present in an enantiomeric excess (ee) of 50 % or more. According to a further preferred embodiment of the present disclosure, the *R*-enantiomer of norepinephrine is present in an enantiomeric excess (ee) of 60 % or more. According to a further preferred embodiment of the present disclosure, the *R*-enantiomer of norepinephrine is present in an enantiomeric excess (ee) of 70 % or more. According to a further preferred embodiment of the present disclosure, the *R*-enantiomer of norepinephrine is present in an enantiomeric excess (ee) of 80 % or more. According to a further preferred embodiment of the present disclosure, the *R*-enantiomer of norepinephrine is present in an enantiomeric excess (ee) of 90 % or more. According to a further preferred embodiment of the present disclosure, the *R*-enantiomer of norepinephrine is present in an enantiomeric excess (ee) of 95 % or more. According to a further preferred embodiment of the present disclosure, the *R*-enantiomer of norepinephrine is present in an enantiomeric excess (ee) of 98 % or more.

According to another embodiment, the liquid phase comprises norepinephrine or a pharmaceutically acceptable salt thereof in a concentration of from about 0.3 mM to about 12 mM.

According to a preferred embodiment of the present disclosure, the liquid phase comprises norepinephrine or pharmaceutically acceptable salt thereof in a concentration of from about 0.5 mM to about 10 mM. According to another preferred embodiment of the present disclosure, the liquid phase comprises norepinephrine or pharmaceutically acceptable salt thereof in a concentration of from about 0.59 mM to about 8 mM. According to a further preferred embodiment of the present disclosure, the liquid phase comprises norepinephrine, or pharmaceutically acceptable salt thereof, in a concentration of from 0.59 mM to about 6 mM.

According to a further preferred embodiment of the present disclosure, the liquid phase comprises norepinephrine, or pharmaceutically acceptable salt thereof in a concentration of about 0.59 mM. Such concentration corresponds to a formulation comprising 0.1 mg/mL of norepinephrine free base.

According to a further preferred embodiment of the present disclosure, the liquid phase comprises norepinephrine, or pharmaceutically acceptable salt thereof, in a concentration of about 1.18 mM. Such concentration corresponds to a formulation comprising 0.2 mg/mL of norepinephrine free base.

According to a further preferred embodiment of the present disclosure, the liquid phase comprises norepinephrine, or pharmaceutically acceptable salt thereof, in a concentration of about 5.91 mM. Such concentration corresponds to a formulation comprising 1.0 mg/mL of norepinephrine free base.

Said concentrations of 0.59 mM and 1.18 mM of norepinephrine are particularly suitable for direct administration, such as intravenous administration, to a patient without prior dilution. On the other hand, formulations comprising 5.91 mM of norepinephrine or a pharmaceutically acceptable salt thereof may be conveniently used as concentrates which are diluted before administration. However, it has been frequently found in the prior art that diluted formulations of norepinephrine are particularly difficult to stabilize. In other words, diluted formulations of norepinephrine, or a pharmaceutically acceptable salt thereof, are especially susceptible to undergo decomposition of the active ingredient. Nevertheless, the inventors of the present disclosure have surprisingly found that diluted liquid formulations of norepinephrine, or a pharmaceutically acceptable salt thereof, are particularly stable over long term storage as shown in examples 1-5 of the present disclosure.

According to another preferred embodiment of the present disclosure, the liquid phase comprises a pharmaceutically acceptable salt of norepinephrine. According to another preferred embodiment of the present disclosure, said pharmaceutically acceptable salt of norepinephrine is norepinephrine tartrate monohydrate (CAS number 339091-66-6). According to another preferred embodiment of the present disclosure, said pharmaceutically acceptable salt of norepinephrine is norepinephrine hydrochloride (CAS number 329-56-6). According to a further preferred embodiment of the present disclosure, said pharmaceutically acceptable salt of norepinephrine is norepinephrine bitartrate (CAS number 108341-18-0).

According to the present disclosure, the liquid phase comprises a buffering system consisting of citric acid and a pharmaceutically acceptable salt thereof. According to a preferred embodiment of the present disclosure, said pharmaceutically acceptable salt of citric acid is sodium citrate.

It is understood that the beneficial effects on the stabilization of norepinephrine, or a pharmaceutically acceptable salt thereof, conferred by said buffering system is the most essential finding of the present disclosure. It has been surprisingly found that a liquid phase comprising said buffering system provides for advantageous stability of norepinephrine. In other words, the specific buffering system allows the preparation of ready-to-inject formulations of norepinephrine with storage stability of 1 year or more, such as 18 months, as evidenced in examples 4 and 5.

According to one embodiment of the present disclosure, the liquid phase comprises the buffering system in a concentration in the range of from about 1 mM to about 30 mM. In other words, the buffering system is present in a concentration in the range of from about 1 mM to about 30 mM. According to a preferred embodiment of the present disclosure, the buffering system is present in the liquid phase in a concentration of from about 3 mM to about 25 mM. According to another preferred embodiment of the present disclosure, the buffering system is present in the liquid phase in a concentration of from about 5 mM to about 20 mM. According to another preferred embodiment of the present disclosure, the buffering system is present in the liquid phase in a concentration of from about 7 mM to about 15 mM. According to another preferred embodiment of the present disclosure, the buffering system is present in the liquid phase in a concentration of from about 8 mM to about 12 mM. According to a further preferred embodiment of the present disclosure, the buffering system is present in the liquid phase in a concentration of about 10 mM.

Although the presence of buffering systems especially at low pH values may be associated with discomfort at the injection site upon administration, the inventors of the present disclosure have found that the specifically low concentrations of the specific buffering system according to the present disclosure are not only suitable to stabilize norepinephrine in liquid formulations, as evidenced in examples 4 and 5, but also are well tolerable.

According to the present disclosure, the liquid phase comprises a tonicity agent. According to a preferred embodiment of the present disclosure, the tonicity agent is sodium chloride, or glucose, or mannitol, preferably sodium chloride or mannitol, more preferably sodium chloride. According to another preferred embodiment of the present disclosure, the tonicity agent is sodium chloride. According to another preferred embodiment of the present disclosure, the tonicity agent is glucose. According to another preferred embodiment of the present disclosure, the tonicity agent is mannitol. According to a further preferred embodiment of the present disclosure, the tonicity agent is sodium chloride.

The inventors of the present disclosure have surprisingly found that the choice of the tonicity agent is particularly advantageous for the stability of norepinephrine in liquid formulations. As shown in example 1, mannitol and NaCl, in particular, show the best stabilizing effects on norepinephrine.

According to a preferred embodiment of the present disclosure, the liquid phase does not contain sodium sulphite. According to a further preferred embodiment of the present disclosure, the liquid phase does not contain a salt of sulphite.

According to another preferred embodiment of the present disclosure, the liquid phase does not contain sodium bisulphite. According to another preferred embodiment of the present disclosure, the liquid phase does not contain a bisulphite salt.

According to another preferred embodiment of the present disclosure, the liquid phase does not contain sodium metabisulphite. According to another preferred embodiment of the present disclosure, the liquid phase does not contain potassium metabisulphite. According to a further preferred embodiment of the present disclosure, the liquid phase does not contain a salt of metabisulphite.

According to a further preferred embodiment of the present disclosure, the liquid phase does not contain a pharmaceutically acceptable sulphite salt. According to a further preferred embodiment of the present disclosure, the liquid phase does not contain a sulphite salt.

According to another preferred embodiment of the present disclosure, the liquid phase does not contain antioxidants selected from the group consisting of butylated hydroxyl anisole, ascorbic acid, sodium ascorbate, propyl gallate, vitamin E, alpha-tocopherol, N-acetyl cysteine, cysteine, pharmaceutically acceptable sulphite salts, and mixtures thereof.

According to a further preferred embodiment of the present disclosure, the liquid phase does not contain antioxidants.

The inventors of the present disclosure have surprisingly found that antioxidant-free, liquid formulations of norepinephrine, or a pharmaceutically acceptable salt thereof, show a surprisingly high stability as evidenced in examples 4 and 5. Even more strikingly, the presence of antioxidants was found to even promote the degradation of norepinephrine and the formation of unknown impurities in the formulations, especially upon thermal sterilization. This is shown in examples 2 and 3. This is very surprising as the prior art teaches to use antioxidant compounds to protect norepinephrine or its pharmaceutically acceptable salt against chemical decomposition reactions. In other words, the finding of the present disclosure is against the technical prejudice established in the field.

According to another preferred embodiment of the present disclosure, the liquid phase does not contain ethylenediaminetetraacetic acid (EDTA), or a pharmaceutically acceptable salt thereof. According to another preferred embodiment of the present disclosure, the liquid phase does not contain gluconic acid, or a pharmaceutically acceptable salt thereof, or derivative thereof, such as a gluconolactone. According to a further preferred embodiment of the present disclosure, the liquid phase does not contain ethylenediaminetetraacetic acid, edetic acid, disodium edetate dihydrate, disodium EDTA, edetate trisodium, edetate sodium, edetate calcium disodium, pentasodium pentetate, di-potassium edetate, dipotassium EDTA, diethylenetriamine pentaacetic acid, or mixtures thereof.

According to another preferred embodiment of the present disclosure, the liquid phase does not contain gluconic acid, or a pharmaceutically acceptable salt thereof, or a derivative thereof. According to a further preferred embodiment of the present disclosure, the liquid phase does not contain a gluconolactone.

According to a further preferred embodiment of the present disclosure, the liquid phase does not contain any chelating agent other than citric acid or a pharmaceutically acceptable salt thereof.

According to another preferred embodiment of the present disclosure, the liquid phase has an osmolarity in the range of from about 260 mOsm/kg to about 320 mOsm/kg. According to a further preferred embodiment of the present disclosure, the osmolarity is determined according to European Pharmacopeia (Ph. Eur.; Monograph Identifying Code "01/2008:0285 corrected 9.3").

According to another preferred embodiment of the present disclosure, the content of norepinephrine, or a pharmaceutically acceptable salt thereof, is at least 95 % (n/n) after storing the container for 12 months or more at room temperature based on the initial content of norepinephrine, or a pharmaceutically acceptable salt thereof. According to another preferred embodiment of the present disclosure, the content of norepinephrine, or a pharmaceutically acceptable salt thereof, is at least 96 % (n/n) after storing the container for 12 months or more at room temperature based on the initial content of norepinephrine or a pharmaceutically acceptable salt thereof. According to another preferred embodiment of the present disclosure, the content of norepinephrine, or a pharmaceutically acceptable salt thereof, is at least 97 % (n/n) after storing the container for 12 months or more at room temperature based on the initial content of norepinephrine or a pharmaceutically acceptable salt thereof. According to a further preferred embodiment of the present disclosure, the content of norepinephrine, or a pharmaceutically acceptable salt thereof, is at least 98 % (n/n) after storing the container for 12 months or more at room temperature based on the initial content of norepinephrine or a pharmaceutically acceptable salt thereof.

According to another preferred embodiment of the present disclosure, the content of norepinephrine, or a pharmaceutically acceptable salt thereof, is at least 95 % (n/n) after storing the container for 18 months or more at room temperature based on the initial content of norepinephrine or a pharmaceutically acceptable salt thereof. According to another preferred embodiment of the present disclosure, the content of norepinephrine, or a pharmaceutically acceptable salt thereof, is at least 96 % (n/n) after storing the container for 18 months or more at room temperature based on the initial content of norepinephrine or a pharmaceutically acceptable salt thereof. According to another preferred embodiment of the present disclosure, the content of norepinephrine, or a pharmaceutically acceptable salt thereof, is at least 97 % (n/n) after storing the container for 18 months or more at room temperature based on the initial content of norepinephrine, or a pharmaceutically acceptable salt thereof. According to a further preferred embodiment of the present disclosure, the content of norepinephrine, or a pharmaceutically acceptable salt thereof, is at least 98 % (n/n) after storing the container for 18 months or more at room temperature based on the initial content of norepinephrine or a pharmaceutically acceptable salt thereof.

According to another preferred embodiment of the present disclosure, the content of norepinephrine, or a pharmaceutically acceptable salt thereof, is at least 95 % (n/n) after storing the container for 4 months or more at 40 °C and 75 % relative humidity based on the initial content of norepinephrine, or a pharmaceutically acceptable salt thereof. According to another preferred embodiment of the present disclosure, the content of norepinephrine, or a pharmaceutically acceptable salt thereof, is at least 96 % (n/n) after storing the container for 4 months or more at 40 °C and 75 % relative humidity based on the initial content of norepinephrine, or a pharmaceutically acceptable salt thereof. According to another preferred embodiment of the present disclosure, the content of norepinephrine, or a pharmaceutically acceptable salt thereof, is at least 97 % (n/n) after storing the container for 4 months or more at 40 °C and 75 % relative humidity based on the initial content of norepinephrine or a pharmaceutically acceptable salt thereof. According to a further preferred embodiment of the present disclosure, the content of norepinephrine, or a pharmaceutically acceptable salt thereof, is at least 98 % (n/n) after storing the container for 4 months or more at 40 °C and 75 % relative humidity based on the initial content of norepinephrine, or a pharmaceutically acceptable salt thereof.

According to a further preferred embodiment of the present disclosure, the content of norepinephrine, or a pharmaceutically acceptable salt thereof, such as the initial content of norepinephrine, or a pharmaceutically acceptable salt thereof, or the content of norepinephrine, or a pharmaceutically acceptable salt thereof, after storing the container for a time at a temperature is determined by high performance liquid chromatography according to the British Pharmacopoeia (BP) 2021, or according to the European Pharmacopoeia (Ph. Eur.; Monograph Identifying Code "01/2008:0285 corrected 9.3"), or according to Assay (I) as described herein, or according to Assay (III) as described herein. It is preferred to use Assay (III) in connection with the container of the present disclosure.

The gas phase

According to the present disclosure, the gas phase has an oxygen content of 0.2 % (V/V) or more based on the total volume of the gas phase.

According to a preferred embodiment of the present disclosure, the gas phase has an oxygen content of about 4.5 % (V/V) or less. According to another preferred embodiment of the present disclosure, the gas phase has an oxygen content of about 4.3 % (V/V) or less. According to another preferred embodiment of the present disclosure, the gas phase has an oxygen content of about 4 % (V/V) or less. According to another preferred embodiment of the present disclosure, the gas phase has an oxygen content of about 3.9 % (V/V) or less. According to another preferred embodiment of the present disclosure, the gas phase has an oxygen content of about 3.8 % (V/V) or less. According to another preferred embodiment of the present disclosure, the gas phase has an oxygen content of about 3.7 % (V/V) or less. According to a further preferred embodiment of the present disclosure, the gas phase has an oxygen content of about 3.5 % (V/V) or less. According to another preferred embodiment of the present disclosure, the gas phase has an oxygen content of about 3.3 % (V/V) or less. According to another preferred embodiment of the present disclosure, the gas phase has an oxygen content of about 3.2 % (V/V) or less. According to another preferred embodiment of the present disclosure, the gas phase has an oxygen content of about 3.0 % (V/V) or less. According to another preferred embodiment of the present disclosure, the gas phase has an oxygen content of about 2.9 % (V/V) or less. According to another preferred embodiment of the present disclosure, the gas phase has an oxygen content of about 2.8 % (V/V) or less. According to another preferred embodiment of the present disclosure, the gas phase has an oxygen content of about 2.7 % (V/V) or less. According to another preferred embodiment of the present disclosure, the gas phase has an oxygen content of about 2.5 % (V/V) or less.

According to another preferred embodiment of the present disclosure, the gas phase has an oxygen content of about 0.5 % (V/V) or more. According to another preferred embodiment of the present disclosure, the gas phase has an oxygen content of about 0.75 % (V/V) or more. According to another preferred embodiment of the present disclosure, the gas phase has an oxygen content of about 0.8 % (V/V) or more. According to another preferred embodiment of the present disclosure, the gas phase has an oxygen content of about 0.9 % (V/V) or more. According to another preferred embodiment of the present disclosure, the gas phase has an oxygen content of about 1 % (V/V) or more. According to another preferred embodiment of the present disclosure, the gas phase has an oxygen content of about 1.1 % (V/V) or more. According to another preferred embodiment of the present disclosure, the gas phase has an oxygen content of about 1.2 % (V/V) or more. According to another preferred embodiment of the present disclosure, the gas phase has an oxygen content of about 1.25 % (V/V) or more. According to another preferred embodiment of the present disclosure, the gas phase has an oxygen content of about 1.3 % (V/V) or more. According to another preferred embodiment of the present disclosure, the gas phase has an oxygen content of about 1.4 % (V/V) or more. According to a further preferred embodiment of the present disclosure, the gas phase has an oxygen content of about 1.5 % (V/V) or more. According to another preferred embodiment of the present disclosure, the gas phase has an oxygen content of about 1.6 % (V/V) or more. According to another preferred embodiment of the present disclosure, the gas phase has an oxygen content of about 1.7 % (V/V) or more. According to another preferred embodiment of the present disclosure, the gas phase has an oxygen content of about 1.8 % (V/V) or more. According to another preferred embodiment of the present disclosure, the gas phase has an oxygen content of about 1.9 % (V/V) or more. According to another preferred embodiment of the present disclosure, the gas phase has an oxygen content of about 2 % (V/V) or more. According to another preferred embodiment of the present disclosure, the gas phase has an oxygen content of about 2.1 % (V/V) or more. According to another preferred embodiment of the present disclosure, the gas phase has an oxygen content of about 2.2 % (V/V) or more. According to another preferred embodiment of the present disclosure, the gas phase has an oxygen content of about 2.4 % (V/V) or more. According to another preferred embodiment of the present disclosure, the gas phase has an oxygen content of about 2.5 % (V/V) or more.

According to another preferred embodiment of the present disclosure, the gas phase has an oxygen content of from about 0.2 % (V/V) to about 5.0 % (V/V). According to another preferred embodiment of the present disclosure, the gas phase has an oxygen content of from about 0.5 % (V/V) to about 4.0 % (V/V). According to another preferred embodiment of the present disclosure, the gas phase has an oxygen content of from about 1 % (V/V) to about 4.0 % (V/V). According to another preferred embodiment of the present disclosure, the gas phase has an oxygen content of from about 1.5 % (V/V) to about 4.0 % (V/V). According to a further preferred embodiment of the present disclosure, the gas phase has an oxygen content of from about 1.5 % (V/V) to about 3.5 % (V/V). According to a further preferred embodiment of the present disclosure, the gas phase has an oxygen content of from about 1.8 % (V/V) to about 3.5 % (V/V). According to a further preferred embodiment of the present disclosure, the gas phase has an oxygen content of from about 1.9 % (V/V) to about 3.5 % (V/V). According to a further preferred embodiment of the present disclosure, the gas phase has an oxygen content of from about 2.0 % (V/V) to about 3.5 % (V/V). According to a further preferred embodiment of the present disclosure, the gas phase has an oxygen content of from about 1.5 % (V/V) to about 1.9 % (V/V).

It is understood that the oxygen content of the gas phase may also be expressed as % of oxygen partial pressure $p_{oxy}$ based on the atmospheric oxygen partial pressure $p_{a,oxy}$. As used herein, the atmospheric oxygen partial pressure $p_{a,oxy}$ is 213 mbar, i.e. 21 % (oxygen content of air) of 1013 mbar (atmospheric pressure $p_a$).

In other words, according to one embodiment of the present disclosure, the gas phase has a $p_{oxy}$ of about 1.0 % or more based on the atmospheric oxygen partial pressure $p_{a,oxy}$. According to a preferred embodiment of the present disclosure, the gas phase has a $p_{oxy}$ of about 2.5 % or more based on the atmospheric oxygen partial pressure $p_{a,oxy}$. According to another preferred embodiment of the present disclosure, the gas phase has a $p_{oxy}$ of about 3.6 % or more based on the atmospheric oxygen partial pressure $p_{a,oxy}$. According to another preferred embodiment of the present disclosure, the gas phase has a $p_{oxy}$ of about 4.8 % or more based on the atmospheric oxygen partial pressure $p_{a,oxy}$. According to another preferred embodiment of the present disclosure, the gas phase has a $p_{oxy}$ of about 5.9 % or more based on the atmospheric oxygen partial pressure $p_{a,oxy}$. According to a further preferred embodiment of the present disclosure, the gas phase has a $p_{oxy}$ of about 7.1 % or more based on the atmospheric oxygen partial pressure $p_{a,oxy}$.

According to another preferred embodiment of the present disclosure, the gas phase has a $p_{oxy}$ of about 21.4 % or less based on the atmospheric oxygen partial pressure $p_{a,oxy}$. According to another preferred embodiment of the present disclosure, the gas phase has a $p_{oxy}$ of about 19.0 % or less based on the atmospheric oxygen partial pressure $p_{a,oxy}$. According to a further preferred embodiment of the present disclosure, the gas phase has a $p_{oxy}$ of about 16.7 % or less based on the atmospheric oxygen partial pressure $p_{a,oxy}$.

According to another preferred embodiment of the present disclosure, the gas phase has a $p_{oxy}$ of from about 1.0 % to about 23.8 % based on the atmospheric oxygen partial pressure $p_{a,oxy}$. According to another preferred embodiment of the present disclosure, the gas phase has a $p_{oxy}$ of from about 4.8 % to about 19.0 % based on the atmospheric oxygen partial pressure $p_{a,oxy}$. According to another preferred embodiment of the present disclosure, the gas phase has a $p_{oxy}$ of from about 7.1 % to about 16.7 % based on the atmospheric oxygen partial pressure $p_{a,oxy}$.

It is understood that the rest of the gas phase, i.e. the part of the gas phase that is not oxygen, may be any other gas. According to a preferred embodiment of the present disclosure, the gas phase further comprises an inert gas. According to a further preferred embodiment of the present disclosure, the gas phase further comprises nitrogen. According to a further preferred embodiment of the present disclosure, the gas phase further comprises argon.

The inventors of the present disclosure have surprisingly found that the content of oxygen in the gas phase can be as high as 4 %(V/V). Yet, the norepinephrine or pharmaceutically acceptable salt thereof comprised in the liquid phase is stable and does not decompose, especially does not oxidize. This is shown in examples 4 and 5.

This is particularly surprising because the prior art teaches to keep the amount of oxygen in a container as low as possible to prevent oxidation of norepinephrine. Nevertheless, the inventors of the present disclosure have found - against the technical prejudice in the field - that ready-to-inject formulations of norepinephrine or a pharmaceutically acceptable salt thereof are stable even in the presence of oxygen in the container, when the specific buffering system according to the present disclosure is used.

Furthermore, it is understood that containers having a high oxygen content in the gas phase are more convenient and cheaper to produce.

The method of manufacture

According to a second aspect, the present disclosure relates to a method for manufacturing a container according to the comprising the steps of

a) dissolving the tonicity agent, buffering system and norepinephrine or pharmaceutically acceptable salt thereof in water;
b) adjusting the pH of the solution obtained from step a);
c) filtering the resulting norepinephrine solution obtained from step b);
d) filling the solution obtained from step c) into a container; and
e) flushing said container with nitrogen to adjust the oxygen content in the gas phase;
wherein at least one of steps step a) to d) is carried out under an atmosphere comprising at least 1 % (V/V) of oxygen.

According to a preferred embodiment, the water used in step a) of the method according to the present disclosure is deoxygenated. According to a preferred embodiment, the water used in step a) of the method according to the present disclosure is degassed. It is understood that deoxygenated water or degassed water can be prepared by flushing the water with an inert gas, such as nitrogen, preferably for a time of at least 30 min.

According to another preferred embodiment of the present disclosure, at least one of steps a) to d) is carried out under an atmosphere comprising of from about 1 % (V/V) to about 22 % (V/V) of oxygen. According to another preferred embodiment of the present disclosure, at least one of steps a) to d) is carried out under an atmosphere of air. According to another preferred embodiment of the present disclosure, at least one of steps a) to d) is carried out under an atmosphere with reduced oxygen content. As used herein, the term "atmosphere with reduced oxygen content" refer to an atmosphere comprising at least 1 % (V/V) of oxygen and 17 % (V/V) or less, preferably 15 % (V/V) or less, more preferably 12 % (V/V) or less, yet more preferably 10 % (V/V) or less, most preferably 6 % (V/V) or less of oxygen.

It is understood that the method of manufacture according to the present disclosure does not require the strict exclusion of oxygen. Therefore, the method according to the present disclosure can be performed conveniently and cost efficiently. At the same time, the formulation of norepinephrine are highly stable upon storage despite the presence of oxygen in the gas phase of the container.

According to another preferred embodiment of the present disclosure, step a) is carried out under an atmosphere comprising at least 1 % (V/V) of oxygen. According to another preferred embodiment of the present disclosure, step b) is carried out under an atmosphere comprising at least 1 % (V/V) of oxygen. According to another preferred embodiment of the present disclosure, step c) is carried out under an atmosphere comprising at least 1 % (V/V) of oxygen. According to another preferred embodiment of the present disclosure, step d) is carried out under an atmosphere comprising at least 1 % (V/V) of oxygen. According to another preferred embodiment of the present disclosure, steps a) and b) are carried out under an atmosphere comprising at least 1 % (V/V) of oxygen. According to another preferred embodiment of the present disclosure, steps a) to c) are carried out under an atmosphere comprising at least 1 % (V/V) of oxygen. According to another preferred embodiment of the present disclosure, steps a) to d) are carried out under an atmosphere comprising at least 1 % (V/V) of oxygen.

According to a further preferred embodiment of the present disclosure, the oxygen content of the gas phase comprised in the container according to the first aspect of the present disclosure is adjusted in step e). In other words, the gas phase comprised in the container contains the atmosphere under which the container is filled in step d). Then, the oxygen content of the gas phase is adjusted by convenient flushing with an inert gas, such as nitrogen, to adjust the oxygen content in the gas phase according to the first aspect of the present disclosure.

According to a preferred embodiment of the present disclosure, the method further comprises the step f) of hermetically sealing of the container obtained from step d). It is understood that step f) is carried out under an atmosphere comprising at least 1 % (V/V) of oxygen. According to another preferred embodiment of the present disclosure, step f) is carried out under an atmosphere comprising at least 1 % (V/V) of oxygen.

It is further understood that the content of dissolved oxygen on the one hand is to be distinguished from the oxygen content of the atmosphere and the oxygen content of the gas phase in the container on the other hand during the method of manufacture according to the present disclosure. In other words, while the method of manufacture, specifically steps a) to c) may preferably be performed with deoxygenated or degassed water, this does not change the oxygen content in the gas phase of the container.

During the method of manufacture, specifically during steps a) to c), the oxygen content in the atmosphere of the tank can be conveniently controlled. Therefore, the amount of dissolved oxygen in the liquid does not change during steps a) to c), but is maintained at the industry standard of below 0.1 ppm.

However, it is very difficult and expensive to control the oxygen content of the atmosphere during the filling and sealing of the containers. Therefore, it is a big advantage of the method of the present disclosure that the filling of the containers in step d) and the sealing of the containers in step e) of the method according to the present disclosure are performed under less strict control of the oxygen content in the atmosphere, i.e. steps d) and e) of the method according to the present disclosure are carried out under an atmosphere comprising at least 1 % (V/V) of oxygen. Therefore, the oxygen content of the gas phase comprised in the container is mainly determined by the oxygen content of the atmosphere during steps d) and e) of the method according to the second aspect of the present disclosure.

After filling and sealing the container, an equilibrium between the oxygen content in the gas phase comprised in the container, and the dissolved oxygen of the liquid phase is established. In other words, the dissolved oxygen in the liquid phase increases within the first minutes or hours of storage while the equilibrium is established. Said equilibrium follows the law of Henry. Since the oxygen in the gas phase is present in large excess as compared to the dissolved oxygen of the liquid phase, the concentration of oxygen in the gas phase does not change considerable while the equilibrium is established. However, the amount of dissolved oxygen in the liquid phase increases considerably from below 0.1 ppm to a value depending on the amount of oxygen comprised in the gas phase.

In other words, the amount of dissolved oxygen in the liquid phase which is the main promoter of norepinephrine degradation is inextricably linked through the law of Henry.

According to another preferred embodiment of the present disclosure, the method further comprises sterilizing the container.

According to another preferred embodiment of the present disclosure, sterilizing the container is a thermal sterilization. According to a further preferred embodiment, sterilizing the container is carried out at a temperature of about 111.8 °C and for a time of at least about 50 min. According to a further preferred embodiment, sterilizing the container is carried out at a temperature of about 111.8 °C and for a time of at least about 75 min, preferably for about 75 min. According to a further preferred embodiment of the present disclosure, sterilizing the container is performed at a temperature of about 121 °C and for a time of at least about 8 min. According to a further preferred embodiment, sterilizing the container is carried out at a temperature of about 121 °C and for a time of at least about 15 min, preferably for about 15 min.

Definitions and general embodiments

As used herein, the term "antioxidants" refers to a chemical compound that inhibits oxidation of norepinephrine by undergoing a chemical reaction with a free radical or other reactive species in the liquid phase.

As used herein, the term "initial content of norepinephrine or a pharmaceutically acceptable salt thereof" refers to the amount of norepinephrine, or a pharmaceutically salt thereof, directly after manufacture of a container according to the present disclosure. In one embodiment, said content may be determined by high performance liquid chromatography according to the British Pharmacopoeia (BP) 2021. In another embodiment, said content may be determined by high performance liquid chromatography according to the European Pharmacopoeia (Ph. Eur.; Monograph Identifying Code "01/2008:0285 corrected 9.3"). According to a preferred embodiment, said content may be determined by high performance liquid chromatography according to Assay (I) as described herein.

As used herein, the "enantiomeric excess (ee)" is determined by Assay (II).

As used herein, the oxygen content of the gas phase is measured directly after manufacture of the container according to the present disclosure. As used herein, the oxygen content of the gas phase is measured with a non-destructive oxygen analyzer, such as the non-destructive gas analyzer FMS-760 or the oxygen meter Microx 4.

As used herein, the concentration of the buffering system refers to the sum of concentrations of citric acid (also referred to as *c(citric acid)*) and the pharmaceutically acceptable salt thereof (also referred to as *c(pharmaceutically acceptable salt of citric acid)*). In other words, the concentration of the buffering system (also referred to as c(buffering system) is calculated as:

*c(buffering system) = c(citric acid) + c(pharmaceutically acceptable salt of citric acid).*

The concentration of citric acid and the pharmaceutically acceptable salt thereof can be determined gravimetrically or by titration, or by calculation using the equation of Henderson-Hasselbalch.

As used herein, the amount of dissolved oxygen (DO) in the liquid phase may be calculated by Henry's law:

$$PO\ [ppm] = \frac{concentration\ of\ O_2\ (gas\ phase)[\%]}{100} \cdot \frac{1}{k_{H,cp}} \cdot 32\ \frac{g}{mol} \cdot 1000$$

with $k_{H,cp}$ of oxygen being 769.2 L*atm/mol.

Within the present invention, the term "chelating agent(s)" shall mean any chelating agent known in the art which is added to a solution of noradrenaline of a pharmaceutically acceptable salt thereof in order to protect noradrenaline from degradation, with the exception of citric acid or pharmaceutically acceptable salts thereof. Examples of chelating agents include in particular metal ion chelators, such as EDTA, EGTA, DTPA and the like. It is understood that the pharmaceutically acceptable anion of the pharmaceutically acceptable salt of norepinephrine, such as in the tartrate/bitartrate salt of noradrenaline, is not considered a chelating agent within the present disclosure. When a formulation does not contain chelating agents in this disclosure, it is understood that the formulation could be defined as lacking any chelating agent as that term is traditionally used in the art, as described, for example, in Rowe et al., Handbook of Pharmaceutical Excipients, 6th Edition 2009. Alternatively, it will be understood that the formulation could be defined as lacking edetic acid or any salt thereof such as disodium edetate (collectively "EDTA").

In the sense of the present disclosure, neither citric acid nor a pharmaceutically acceptable salt of citric acid is a chelating agent.

As used herein, the osmolarity is determined according to the European Pharmacopeia (Monograph Identifying Code "01/2008:0285 corrected 9.3").

Where the term "comprising" is used in the present description and claims, it does not exclude other elements. For the purposes of the present disclosure, the term "consisting of" is considered to be a preferred embodiment of the term "comprising". If a group is defined to comprise at least a certain number of embodiments herein, this is also to be understood to disclose a group, which preferably consists only of these embodiments. Furthermore, if a composition is defined using the term "comprising", it may additionally comprise other elements not explicitly listed, however, not further amounts of an element listed. As such, if, e.g., an anti-fungal composition comprises caspofungin, or a pharmaceutically acceptable salt thereof in an amount of 100 $\mu$mol, said anti-fungal-composition may comprise elements other than caspofungin, or a pharmaceutically acceptable salt thereof, however, not additional amounts of caspofungin, or a pharmaceutically acceptable salt thereof, thereby exceeding the amount of 100 $\mu$mol.

The term "about" in conjunction with a numerical value refers to normal deviations of said numerical value. It is to be understood that the term "about" can mean a deviation of $\pm$ 10 %, preferably $\pm$ 5 %, more preferably $\pm$ 2.5 % of said numeric value as indicated.

Assay (I)

For the determination of the amount of norepinephrine, or a pharmaceutically acceptable salt thereof, and the amount of impurities, a 15 minute reversed-phase liquid chromatography method according to the British Pharmacopoeia (version of 2021) is used. For the assay, the liquid phase was used without dilution. The analysis of 20 $\mu$L injected sample is performed on a stainless steel column (100x4.6 mm; packed with end-capped octadecylsilyl silica gel (5 $\mu$m)) maintained at 25 °C. Elution conditions involve an isocratic elution with a mobile phases consisting of 4.0 g of tetramethylammonium hydrogen sulfate, 1.1 g of sodium heptanesulfonate, and 2 mL of 0.1 M disodium edeteate in 950 mL of water and 50 mL of methanol. The pH is adjusted to 3.5 with 1 M sodium hydroxide.

Norepinephrine and its degradation products or impurities are detected by UV detection at 205 nm and the respective peak areas are calculated.

The amount of norepinephrine and impurities in %, such as % (n/n), is performed by external calibration using a working standard of norepinephrine.

Assay (II)

For the determination of the amount of *R*-norepinephrine or a pharmaceutically acceptable salt thereof, a 10 minute chiral liquid chromatography method is used. Said method is referred to as Assay (II). For the assay, the liquid phase was used without dilution. The analysis of 10 μL injected sample is performed on a chiral CBH column (5 μm 100x4.0 mm) maintained at 10 °C with a flow rate of 0.9 mL/min. Elution conditions involve an isocratic elution with a mobile phases consisting of 95 % (V/V) of buffer A and 5 % (V/V) of 2-propanole. Buffer A is prepared by dissolving 1.2 g of $NaH_2PO_4$ in 900 mL of water. The pH of said solution is adjusted to 5.8 with 1 M NaOH and diluted to 1000 mL with water. Then, 18.6 mg of disodium edeteate is added to provide buffer A.

*R*-Norepinephrine and *S*-norepinephrine are detected by UV detection at 280 nm and the respective peak areas are calculated.

The amount of *R*-norepinephrine and *S*-enantiomer in %, such as % (n/n), is performed by external calibration using a working standard of the respective norepinephrine.

Assay (III)

For the determination of the amount of norepinephrine, or a pharmaceutically acceptable salt thereof, and the amount of impurities, a 30 minute reversed-phase liquid chromatography method according to the European Pharmacopoeia (Monograph Identifying Code "01/2008:0285 corrected 9.3") is used. For the assay, the liquid phase was used without dilution. The analysis of 3 μL injected sample (1.18 mM of norepinephrine or a pharmaceutically acceptable salt thereof), or 6 μL injected sample (0.59 mM of norepinephrine or a pharmaceutically acceptable salt thereof), respectively, is performed on a stainless steel column (100x4.6 mm; packed with end-capped octadecylsilyl silica gel) maintained at 25 °C. Elution conditions involve a gradient elution with a mobile phases consisting of mobile phase A (0.5 g of sodium heptanesulfonate R in 1000 mL; pH adjusted to 2.2 with phosphoric acid R) and mobile phase B (0.25 g of sodium heptanesulfonate R in 500 mL; add 500 mL of acetonitrile HPLC grade; pH adjusted to 2.4 with phosphoric acid R). The gradient is

| Time [min] | Mobile phase A [% (V/V)] | Mobile phase B [% (V/V)] | Flow rate [mL/min] |
|---|---|---|---|
| 0 - 2.0 | 98 | 2 | 1.5 |
| 2.0 - 17.0 | 98 → 70 | 2 → 30 | 1.5 |
| 17.0 - 24.0 | 70 → 50 | 30 → 50 | 1.5 |
| 24.0 - 24.1 | 50 → 0 | 50 → 100 | 1.5 → 4.0 |
| 24.1 - 28.0 | 0 | 100 | 4.0 |
| 28.0 - 28.1 | 0 → 98 | 100 → 2 | 4.0 |
| 28.1 - 30.0 | 98 | 2 | 4.0 → 1.5 |

Norepinephrine and its degradation products or impurities except for impurity F are detected by UV detection at 280 nm and the respective peak areas are calculated. Impurity F is detected at 254 nm and the peak area is calculated.

The amount of norepinephrine and impurities in %, such as % (n/n), is performed by external calibration using a working standard of norepinephrine.

**Examples**

Example 1

To investigate the effect of the tonicity agent on the stability of norepinephrine in a liquid formulation, norepinephrine bitartrate and the respective tonicity agents were dissolved in deoxygenated water (residual oxygen < 0.1 ppm). The resulting solutions were adjusted with 1 N HCl to pH 3.4 and filtered without inert gas current. Then, the solutions were filled into vials manually with an automatic pipette and each vial was degassed with $N_2$. Then, the vials were stepped and capsulated rapidly to result in a gas phase comprising less than 2 % (V/V) of oxygen as determined with a non-destructive oxygen analyzer. The vials were sterilized at 121 °C for 15 min.

The ratio of gas phase to liquid phase in the vial was 0.8 mL of gas phase : 20 mL of liquid phase.

**Table 1** Overview on compositions F1 to F3

| | | F1 | F2 | F3 |
|---|---|---|---|---|
| Active agent | Norepinephrine bitartrate [mg] | 20.0 | 20.0 | 20.0 |

(continued)

|  |  | **F1** | **F2** | **F3** |
|---|---|---|---|---|
| Tonicity agent | NaCl [mg] | 870.0 |  |  |
| | glucose# [mg] |  | 5000.0 |  |
| | mannitol [mg] |  |  | 5070.0 |
| pH adjuster | HCl 1N | q.s. pH 3.4 | q.s. pH 3.4 | q.s. pH 3.4 |
| Solvent | water [ml] | q.s. 100 | q.s. 100 | q.s. 100 |
| oxygen content of gas phase [% (V/V)] | | < 2 | < 2 | < 2 |
| # added as 5500.0 mg of glucose monohydrate. | | | | |

Formulations F1 to F3 were subjected to stability study for 3 to 13 weeks at an elevated temperature of 40 °C. The amount of norepinephrine and impurities was determined according to the norepinephrine injection monograph of the British Pharmacopoeia 2021. Acceptance criteria were: pH in the range of from 3.0 to 4.6; osmolarity in the range of from 270 to 310 mOsmol/kg; norepinephrine 95.0 % to 105 %; adrenaline 1 % or less; total impurities 0.5 % or less; sub-visible particles ≥ 10 $\mu$m of 6000 particles/vial or less; sub-visible particles ≥ 25 $\mu$m of 600 particles/vial or less.

**Table 2** Results of stability studies

|  | **F1** | | **F2** | | **F3** | |
|---|---|---|---|---|---|---|
| storing time [weeks] | 0 | 13 | 0 | 13 | 0 | 13 |
| pH | 3.4 | 3.5 | 3.4 | 3.5 | 3.5 | 3.6 |
| Osmolarity [mOsmol/kg] | 275 | N/A | 296 | N/A | 293 | N/A |
| Noradenaline [% (n/n)] | 100.3 | 98.2 | 99.2 | 96.2 | 99.9 | 97.8 |
| Adrenaline [A%] | N.D. | 0.19 | N.D. | 0.30 | 0.1 | 0.48 |
| total impurities [A%] | < LoQ | < LoQ | < LoQ | 1.4 | < LoQ | 0.57 |
| sub-visible particles ≥10 $\mu$m | 860 | 665 | 217 | 113 | 251 | 6629 |
| sub-visible particles ≥25 $\mu$m | 140 | 21 | 43 | 13 | 65 | 81 |
| Oxygen in gas phase [% (V/V)] | 0.4 | 1.9 | 0.8 | 1.6 | 1.9 | 1.8 |
| Acceptance criteria met? | Yes | Yes | Yes | No | Yes | No |
| LoQ: Limit of quantification (0.1 %); N.D.: not detectable; N/A: not determined. | | | | | | |

As can be concluded from table 2, formulation F1 containing NaCl as tonicity agent shows excellent stability after 13 weeks of storage at 40 °C. In particular, the formation of impurities is especially low.

Furthermore, the formulations were subjected to stability studies at further elevated temperatures to additionally evaluate the influence of the tonicity agent on the stability of norepinephrine in the formulations F1 to F3.

**Table 3** Results of stability studies at further elevated temperatures

| Condition |  | **70 °C** | | **55 °C** | | |
|---|---|---|---|---|---|---|
| storage time [weeks] | 0 | 1 | 3 | 1 | 3 | 5 |
| **F1** Norepinephrine [% (n/n)] | 100.3 | 96.9 | 89.9 | 99.3 | 98.1 | 96.0 |
| **F2** Norepinephrine [% (n/n)] | 99.1 | 93.4 | 79.5 | 98.1 | 97.0 | 93.9 |
| **F3** Norepinephrine [% (n/n)] | 99.9 | 97.5 | 90.8 | 99.1 | 98.8 | 97.5 |

In conclusion, NaCl is the best tonicity agent as it provides sufficient stability of norepinephrine with low formation of impurities, whereas also mannitol and glucose show acceptable stability.

Example 2

To investigate the influence of the presence of antioxidant compounds on the stability of norepinephrine in formulations during a sterilization process, different formulations F4-F7 comprising different antioxidative agents were prepared.

Water was deoxygenated by flowing N$_2$ for 30 min. 90 % of the required amount of said deoxygenated water was placed in a glass vessel and tonicity agent, antioxidant (where applicable), and norepinephrine bitartrate were added. pH was adjusted wit 1 N HCl or 1 N NaOH to pH 3.4. Then, deoxygenated water was added to the final volume.

**Table 4** Overview on compositions F4 to F7

|  | F4 | F5 | F6 | F7 |
|---|---|---|---|---|
| Norepinephrine base [mg] | 10.0 | 10.0 | 10.0 | 10.0 |
| NaCl [mg] | 900.0 | 900.0 | 900.0 | 900.0 |
| L-ascorbic acid [mg] | - | 100.0 | - | - |
| NAC* [mg] | - | - | 100.0 | - |
| L-cysteine [mg] | - | - | - | 100.0 |
| HCl/NaOH 1 N | q.s. pH 3.4 | q.s. pH 3.4 | q.s. pH 3.4 | q.s. pH 3.4 |
| water [mL] |  |  |  |  |
| oxygen content of gas phase [% (V/V)] | < 2 | < 4 | < 4 | < 4 |
| *NAC: N-acetyl-L-cysteine | | | | |

Each solution was filtered through a 0.22 μm Stericup® Darapore PVDF (Cat. No. SCGVU11RE), and the filtered solutions were degassed in a nitrogen current for 30 minutes. 10.0 mL of each of the solutions was filled in a respective 10 mL ampoule under nitrogen flow and sealed.

The oxygen in the gas phase was tested by a non-destructive oxygen analyser. Half of the vials of formulations F4-F7 were subjected to a terminal sterilization treatment at 121 °C for at least 15 min. The other half was not subjected to sterilization.

The amount of norepinephrine and impurities was determined according to Assay (III). Acceptance criteria were: pH in the range of from 3.0 to 4.6; norepinephrine 95.0 % to 105 %; total impurities 0.5 % or less; sub-visible particles ≥ 10 μm of 6000 particles/vial or less; sub-visible particles ≥ 25 μm of 600 particles/vial or less.

**Table 5** Results of stability studies during thermal sterilization treatment

|  | F4 | | F5 | | F6 | | F7 | |
|---|---|---|---|---|---|---|---|---|
| antioxidant | none | | ascorbic acid | | N-acetyl-L-cysteine | | L-cysteine | |
| steriliziation | No | Yes | No | Yes | No | Yes | No | Yes |
| pH | 3.5 | 3.5 | 3.4 | 3.4 | 3.5 | 3.5 | 3.5 | 3.5 |
| norepinephrine [% (n/n)] | 98.9 | 99.1 | 99.2 | 7.4 | 99.4 | 93.2 | 100.3 | 91.1 |
| total impurities [A %] | N.D. | N.D. | N.D. | 3.87 | 1.47 | 8.15 | 1.68 | 12.48 |
| sub-visible particles ≥ 10 μm | - | 471 | - | 1476 | - | - | - | - |
| sub-visible particles ≥ 25 μm | - | 9 | - | 24 | - | - | - | - |
| Oxygen in gas phase [% (V/V)] | - | 1.9 | - | 3.2 | - | 3.9 | - | 4.3 |
| Acceptance criteria met? | Yes | Yes | Yes | No | No | No | No | No |
| N.D.: not detectable; - means not determined. | | | | | | | | |

The presence of antioxidant compounds, such as ascorbic acid, L-cysteine, and N-acetyl-L-cysteine, reduce the stability of norepinephrine upon sterilization. Therefore, it is preferred to prepare formulations of norepinephrine that do not contain antioxidant compounds.

Example 3

To investigate the influence of the presence of antioxidant compounds on the stability of norepinephrine in formulations during a sterilization process, different formulations F9-F11.4 comprising different antioxidative agents were prepared.

For that, non-deoxygenated water was placed in a glass vessel and tonicity agent, antioxidant (where applicable), buffering system and norepinephrine bitartrate were added under an atmosphere of air. pH was adjusted wit 1N HCl or 1N NaOH to pH 3.4. Then, non-deoxygenated water was added to the final volume.

**Table 6** Overview on compositions F9 to F10.2

|  | F9 | F10 | F10.1 | F10.2 |
|---|---|---|---|---|
| Norepinephrine base [mg] | 10.0 | 10.0 | 10.0 | 10.0 |
| NaCl [mg] | 50.0 | 50.0 | 150.0 | 500.0 |
| L-ascorbic acid [mg] | - | 100.0 | 350.0 | - |
| Gluconolactone [mg] | 2500.0 | 2500.0 | 2500.0 | 1250.0 |
| Na ascorbate [mg] | - | - | - | 300 |
| HCl/NaOH 1N | q.s. pH 3.4 | q.s. pH 3.4 | q.s. pH 3.4 | q.s. pH 3.4 |
| water [mL] | q.s. 100 | q.s. 100 | q.s. 100 | q.s. 100 |
| oxygen content of gas phase [% (V/V)] | 21 | 21 | 21 | 21 |

**Table 7** Overview on compositions F11 to F11.4

|  | F11 | F11.1 | F11.2 | F11.3 | F11.4 |
|---|---|---|---|---|---|
| Norepinephrine base [mg] | 10.0 | 10.0 | 10.0 | 10.0 | 10.0 |
| NaCl [mg] | 50.0 | 50.0 | 350.0 | 700.0 | 870.0 |
| Gluconolactone [mg] | 2500.0 | 2500.0 | 1250.0 | 750.0 | - |
| Citric acid [mg] | 300.0 | - | - | - | 98.0 |
| Na citrate [mg] |  | 300.0 | 300.0 | 300.0 | 162.0 |
| HCl/NaOH 1N | q.s. pH 3.4 | q.s. pH 3.4 | q.s. pH 3.4 | q.s. pH 3.4 | q.s. pH 3.4 |
| water [mL] | q.s. 100 | q.s. 100 | q.s. 100 | q.s. 100 | q.s. 100 |
| oxygen content of gas phase [% (V/V)] | 21 | 21 | 21 | 21 | 21 |

Each solution was filtered through a 0.22 $\mu$m Stericup® Darapore PVDF (Cat. No. SCGVU11RE). 20.0 mL of each of the solutions was filled in a respective 20 ml glass vial. Then, the vials were closed with a rubber stopper and a capsule tear-off.

Half of the vials of formulations F9 to F11.4 were subjected to a terminal sterilization treatment at 111.8 °C for at least 75 min. The other half was not subjected to sterilization, but to aseptic filling.

The amount of norepinephrine was determined for each formulation with and without sterilization treatment according to the norepinephrine injection monograph of Assay (I).

**Table 8** Results of stability studies of F9-F11.4 upon thermal sterilization

|  | Composition | Norepinephrine [% (n/n)] Sterilization | | loss on sterilization [% (n/n)] |
|---|---|---|---|---|
|  |  | No | Yes |  |
| F9 | Gluconolactone 25 mg/mL | 93.3 | 89.3 | 4.3 |
| F10 | Gluconolactone 25 mg/mL + Ascorbic acid 1 mg/mL | 97.9 | 89.5 | 8.6 |

(continued)

| | Composition | Norepinephrine [% (n/n)] Sterilization | | loss on sterilization [% (n/n)] |
|---|---|---|---|---|
| | | No | Yes | |
| F10.1 | Gluconolactone 25 mg/mL + Ascorbic acid 3.5 mg/mL | 97.9 | 89.5 | 8.6 |
| F10.2 | Gluconolactone 12.5 mg/mL + sodium ascorbate 3 mg/mL | 99.1 | 94.0 | 5.1 |
| F11 | Gluconolactone 25 mg/mL + citric acid 3 mg/mL | 101.2 | 97.5 | 3.7 |
| F11.1 | Gluconolactone 25 mg/mL + sodium ascorbate 3 mg/mL | 99.1 | 94.8 | 4.3 |
| F11.2 | Gluconolactone 12.5 mg/mL + sodium citrate 3 mg/mL | 99.8 | 97.0 | 2.8 |
| F11.3 | Gluconolactone 7.5 mg/mL + sodium ascorbate 3 mg/mL | 100.1 | 97.3 | 2.8 |
| F11.4 | sodium citrate 1.62 mg/mL + citric acid 0.98 mg/mL | 100.5 | 99.6 | 0.9 |

As already shown in Example 2, the addition of an antioxidant such as ascorbic acid or sodium ascorbate decreases the stability of norepinephrine during sterilization (F10, F10.1, F10.2, F11.1, F11.3). Therefore, antioxidant compounds such as ascorbic acid/sodium ascorbate are incompatible with thermal sterilization. However, the addition of citric acid and sodium citrate results in a certain stabilization (F11-F11.4). Decreasing concentrations of gluconolactone increase stabilization (F11.2, F11.3). Formulation with only citric acid / sodium citrate buffer results in best stabilization (F11.4). Interestingly, the advantageous effect of citric acid / sodium citrate on stability upon terminal sterilization was also present without any control of oxygen, as demonstrated in this study. On the other hand, antioxidants do not increase stability of norepinephrine during thermal sterilization and may even be disadvantageous.

Example 4

Formulation F11.4 of norepinephrine (0.1 mg/mL of norepinephrine free base) was prepared as described above: 1.00 g of norepinephrine bitartrate, 43.50 g of NaCl, 4.9 g of citric acid monohydrate, and 8.10 g of sodium citrate dihydrate were dissolved in deoxygenated MilliQ water. The pH is adjusted to 3.4 with 1 M HCl and deoxygenated water was added to reach a total volume of 5 L.

A part of the vials was thermally sterilized at 111.8 °C for 75 min, the other was produced using aseptic techniques. The samples were stored for 10 weeks at either 25 °C and 60 % relative humidity (rH), or 30 °C and 65 % relative humidity (rH), or 40 °C and 75 % relative humidity (rH) in the dark.

The content of norepinephrine was determined according to Assay (I). The contents of adrenaline, noradrenolone, and unknown impurities were determined according to the method of Assay (III). Furthermore, the amount of other unknown impurities was determined with the method of the European Pharmacopoeia.

Table 9 Results of stability studies of F11.4 under different storage conditions for 10 weeks

| Condition | 25 °C / 60 % rH | | 30 °C / 65 % rH | | 40 °C / 75 % rH | |
|---|---|---|---|---|---|---|
| sterilization | No | Yes | No | Yes | No | Yes |
| Norepinephrine [% (n/n)] | 100.8 | 100.9 | 101.0 | 101.1 | 100.3 | 100.3 |
| Adrenaline [A%] | < LoQ | < LoQ | < LoQ | < LoQ | < LoQ | < LoQ |
| total unknown impurities [A%] | 0.12 | 0.45 | < LoQ | 0.45 | 0.17 | 0.47 |
| oxygen in gas phase [% (V/V)] | 0.8 | 2.0 | 1.8 | 1.1 | 2.7 | 3.1 |
| LoQ: limit of quantification (0.1 %); Noradrenolone levels were below the LoQ . | | | | | | |

From the stability studies is can be concluded that formulation F11.4 is highly stable, even after thermal sterilization. In other words, thermal sterilization is well tolerated.

Example 5

To further investigate the influence of the oxygen content of the gas phase, formulation F11.4 was prepared as described above. The formulation was filled into glass vials under aseptic conditions and the oxygen content of the gas phase was adjusted by flushing the glass vials with nitrogen.

The samples were stored for several weeks at either 25 °C and 60 % relative humidity (rH), or 30 °C and 65 % relative humidity (rH), or 40 °C and 75 % relative humidity (rH), or 55 °C or 70 °C in the dark.

The content of norepinephrine was determined according to the method of the British Pharmacopoeia.

**Table 10** Results of stability studies of F11.4 under different storage conditions

| Storage time [weeks] | 0 | 3 | 7 | 10 | 13 | 18 | 29 | 48 |
|---|---|---|---|---|---|---|---|---|
| at | Norepinephrine assay [% (n/n)]; $O_2$ content in gas phase < 2 % (V/V) | | | | | | | |
| 25 °C / 60 % rH | 100.5 | - | - | 100.8 | - | 101.1 | 99.9 | 100.7 |
| 30 °C / 65 % rH | 100.5 | - | - | 101.0 | 100.9 | 100.8 | 99.7 | 100.5 |
| 40 °C / 75 % rH | 100.5 | - | 100.7 | 100.3 | 100.2 | 99.7 | 97.8 | - |
| 55 °C | 100.5 | 100.4 | - | 98.4 | - | - | - | - |
| 70 °C | 100.5 | 96.0 | 89.4 | | | | | |
| | Norepinephrine assay [% (n/n)]; $O_2$ content in gas phase 3-4 % (V/V) | | | | | | | |
| 25 °C / 60 % rH | 100.5 | - | - | 99.2 | - | 100.5 | 99.3 | 100.0 |
| 30 °C / 65 % rH | 100.5 | - | - | 100.8 | 100.6 | - | 98.9 | 97.7 |
| 40 °C / 75 % rH | 100.5 | - | 100.4 | 100.1 | 99.8 | 98.4 | 97.2 | - |
| 55 °C | 100.5 | 100.1 | 98.3 | 96.0 | - | - | - | - |
| 70 °C | 100.5 | 91.4 | 82.7 | - | - | - | - | - |
| - means not determined | | | | | | | | |

Furthermore, the amount of total impurities was determined with the method of Assay (III).

**Table 11** Amount of impurities formed in F11.4 under different storage conditions

| Storage time [weeks] | 0 | 3 | 7 | 10 | 13 | 18 | 29 | 48 |
|---|---|---|---|---|---|---|---|---|
| at | total impurities (A%); $O_2$ content in gas phase < 2 % (V/V) | | | | | | | |
| 25 °C / 60 % rH | < LoQ | | - | 0.12 | - | < LoQ | < LoQ | < LoQ |
| 30 °C / 65 % rH | < LoQ | - | - | < LoQ | < LoQ | < LoQ | < LoQ | 0.11 |
| 40 °C / 75 % rH | < LoQ | - | < LoQ | 0.17 | < LoQ | < LoQ | 1.47 | - |
| 55 °C | < LoQ | 0.21 | 0.58 | 1.27 | - | - | - | - |
| 70 °C | < LoQ | 4.47 | 10.36 | - | - | - | - | - |
| | total impurities (A%); $O_2$ content in gas phase 3-4 % (V/V) | | | | | | | |
| 25 °C / 60 % rH | < LoQ | - | - | 0.20 | - | < LoQ | < LoQ | < LoQ |
| 30 °C / 65 % rH | < LoQ | - | - | < LoQ | < LoQ | < LoQ | < LoQ | 0.69 |
| 40 °C / 75 % rH | < LoQ | - | < LoQ | 0.15 | 0.12 | 0.37 | 1.81 | - |
| 55 °C | < LoQ | 0.19 | 5.66 | 2.36 | - | - | - | - |

(continued)

| | total impurities (A%); O$_2$ content in gas phase 3-4 % (V/V) | | | | | | | |
|---|---|---|---|---|---|---|---|---|
| 70 °C | < LoQ | 4.78 | 9.71 | - | - | - | - | - |
| - means not determined; < LoQ means below limit of quantification (0.1 %) | | | | | | | | |

For formulation F11.4 with an amount oxygen in the gas phase of less than 2 % (V/V), the total impurities after 29 weeks of storage at 40 °C was 1.5 % and practically no formation of impurities was observed at 30 °C and below. Even with an increased oxygen content in the gas phase of about 3-4 % (V/V), the total impurities after 29 weeks at 40 °C was 1.8 % and practically no formation of impurities at 30 °C and below could be observed.

In conclusion, the formulations according to the present disclosure show high storage stability even at high oxygen contents.

**Claims**

1. A container comprising a liquid phase and a gas phase, wherein

   a) the liquid phase is a pharmaceutical composition comprising

      i) norepinephrine, or a pharmaceutically acceptable salt thereof, in a concentration of from about 0.3 mM to about 12 mM, and
      ii) a buffering system consisting of citric acid and a pharmaceutically acceptable salt thereof, wherein the buffering system is present in a concentration in the range of from 1 mM to 30 mM; and
      iii) at least one tonicity agent; and
      iv) water,

   wherein the liquid phase has a pH in the range of from about 3.0 to about 4.0, and,
   b) the gas phase has an oxygen content of 0.2 % (V/V) or more based on the total volume of the gas phase.

2. The container according to claim 1, wherein the gas phase has an oxygen content

   a) of about 4.5 % (V/V) or less, preferably of about 4 % (V/V) or less, more preferably of about 3.5 % (V/V) or less; and/or
   b) of about 1 % (V/V) or more, preferably of about 1.5 % (V/V) or more; and/or
   c) of from about 0.2 % (V/V) to about 5.0 % (V/V), preferably of from about 1 % (V/V) to about 4.0 % (V/V), most preferably of from about 1.5 % (V/V) to about 3.5 % (V/V);

   each based on the total volume of the gas phase.

3. The container according to claim 1 or 2, wherein the liquid phase has a pH in the range of from about 3.2 to about 3.8, preferably of from about 3.3 to about 3.6, more preferably of from about 3.35 to about 3.45, most preferably about 3.4.

4. The container according to any one of claims 1 to 3, wherein the norepinephrine is the R-enantiomer of norepinephrine, or the S-enantiomer of norepinephrine, or a mixture of R-enantiomer and S-enantiomer of norepinephrine, preferably wherein the norepinephrine is the R-enantiomer of norepinephrine.

5. The container according to any one of claims 1 to 4, wherein the liquid phase comprises norepinephrine, or pharmaceutically acceptable salt thereof, in a concentration of from about 0.5 mM to about 10 mM, preferably of from about 0.59 mM to about 8 mM, more preferably of from 0.59 mM to about 6 mM, most preferably, in a concentration of about 0.59 mM, or of about 1.18 mM or of about 5.91 mM.

6. The container according to any one of claims 1 to 5, wherein

   a) the pharmaceutically acceptable salt of norepinephrine is norepinephrine bitartrate, or norepinephrine tartrate monohydrate, or norepinephrine hydrochloride, preferably wherein the pharmaceutically acceptable salt of norepinephrine is norepinephrine bitartrate; and/or
b) the pharmaceutically acceptable salt of citric acid is sodium citrate.

7.  The container according to any of claims 1 to 6, wherein the tonicity agent is sodium chloride, or glucose, or mannitol, preferably sodium chloride or mannitol, more preferably sodium chloride.

8.  The container according to any of claims 1 to 7, wherein the liquid phase does not contain sodium sulphite, sodium bisulphite, sodium metabisulphite, potassium metabisulphite, or mixtures thereof, preferably wherein the liquid phase does not contain pharmaceutically acceptable sulphite salts; preferably wherein the liquid does not contain antioxidants selected from the group consisting of butylated hydroxyl anisole, ascorbic acid, sodium ascorbate, propyl gallate, vitamin E, alpha-tocopherol, N-acetyl cysteine, cysteine and pharmaceutically acceptable sulphite salts, most preferably the liquid phase does not contain antioxidants.

9.  The container according to any of claims 1 to 8, wherein the liquid phase does not contain

    a) an ethylenediaminetetraacetic acid (EDTA) or pharmaceutically acceptable salt thereof, preferably the liquid phase does not contain ethylenediaminetetraacetic acid, edetic acid, disodium edetate dihydrate, disodium EDTA, edetate trisodium, edetate sodium, edetate calcium disodium, pentasodium pentetate, di-potassium edetate, dipotassium EDTA, diethylenetriamine pentaacetic acid or mixtures thereof; and/or
    b) gluconic acid, or a pharmaceutically acceptable salt thereof, or a derivative thereof, preferably the liquid phase does not contain a gluconolactone.

10. The container according to any one of claims 1 to 9, wherein the liquid phase has an osmolarity in the range of from about 260 mOsm/kg to about 320 mOsm/kg, preferably wherein the osmolarity is determined according to European Pharmacopeia (Monograph Identifying Code "01/2008:0285 corrected 9.3").

11. The container according to any one of claims 1 to 10, wherein

    a) the content of norepinephrine, or a pharmaceutically acceptable salt thereof, is at least 95 % (n/n), preferably at least 96 % (n/n), more preferably at least 97 % (n/n), most preferably at least 98 % (n/n) after storing the container for 18 months or more at room temperature based on the initial content of norepinephrine or a pharmaceutically acceptable salt thereof; and/or
    b) the content of norepinephrine, or a pharmaceutically acceptable salt thereof, is at least 95 % (n/n), preferably at least 96 % (n/n), more preferably at least 97 % (n/n), most preferably at least 98 % (n/n) after storing the container for 4 months or more at 40 °C and 75 % relative humidity based on the initial content of norepinephrine or a pharmaceutically acceptable salt thereof; preferably

    wherein the content of norepinephrine, or pharmaceutically acceptable salt thereof, is determined by high performance liquid chromatography according to the British Pharmacopoeia 2021 or the European Pharmacopoeia (Monograph Identifying Code "01/2008:0285 corrected 9.3"), or Assay (I), or Assay (III).

12. The container according to any one of claims 1 to 11, wherein the container is a prefilled syringe or a sealed container or an IV bag, wherein the sealed container is a glass vial or a glass ampoule.

13. A method for manufacturing a container according to any one of claims 1 to 12 comprising the steps of

    a) dissolving the tonicity agent, buffering system and norepinephrine or pharmaceutically acceptable salt thereof in water;
    b) adjusting the pH of the solution obtained from step a);
    c) filtering the resulting norepinephrine solution obtained from step b);
    d) filling the solution obtained from step c) into a container; and
    e) flushing said container with nitrogen to adjust the oxygen content in the gas phase;

    wherein at least one of steps a) to d) is carried out under an atmosphere comprising at least 1 % (V/V) of oxygen.

14. The method according to claim 13, wherein the method further comprises the step f) hermetically sealing of the container obtained from step e).

15. The method according to claim 13 or 14, wherein the method further comprises sterilizing the container.

Europäisches
Patentamt

European
Patent Office

Office européen
des brevets

# EUROPEAN SEARCH REPORT

**Application Number**

**EP 23 17 6898**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/362175 A1 (HINGORANI TUSHAR [US] ET AL) 17 November 2022 (2022-11-17) * paragraphs [0015], [0029], [0031] – [0033]; claim 1; table 4 * ----- | 1-15 | INV. A61K47/02 A61K9/00 A61K31/137 A61K47/26 A61K47/20 A61K47/12 A61K47/18 |
| | | | TECHNICAL FIELDS SEARCHED (IPC) |
| | | | A61K |

The present search report has been drawn up for all claims

| Place of search | Date of completion of the search | Examiner |
|---|---|---|
| The Hague | 30 October 2023 | Schwald, Claudia |

EPO FORM 1503 03.82 (P04C01)

**ANNEX TO THE EUROPEAN SEARCH REPORT
ON EUROPEAN PATENT APPLICATION NO.**

EP 23 17 6898

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2023

| Patent document cited in search report | Publication date | Patent family member(s) | | Publication date |
|---|---|---|---|---|
| US 2022362175 A1 | 17-11-2022 | CA | 3051467 A1 | 02-08-2018 |
| | | US | 2018214394 A1 | 02-08-2018 |
| | | US | 2018243243 A1 | 30-08-2018 |
| | | US | 2019046473 A1 | 14-02-2019 |
| | | US | 2019046474 A1 | 14-02-2019 |
| | | US | 2019133972 A1 | 09-05-2019 |
| | | US | 2019133973 A1 | 09-05-2019 |
| | | US | 2020230079 A1 | 23-07-2020 |
| | | US | 2022362175 A1 | 17-11-2022 |
| | | US | 2022409558 A1 | 29-12-2022 |
| | | WO | 2018140894 A1 | 02-08-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 339091-66-6
- *CHEMICAL ABSTRACTS,* 329-56-6
- *CHEMICAL ABSTRACTS,* 108341-18-0
- British Pharmacopoeia (BP). 2021

- **ROWE et al.** Handbook of Pharmaceutical Excipients. 2009
- *British Pharmacopoeia,* 2021